# EUROPÄISCHE PATENTSCHRIFT

(11) **EP 0 888 284 B1**
(45) Veröffentlichungstag und Bekanntmachung des Hinweises auf die Patenterteilung: **24.10.2001**
(21) Anmeldenummer: 97907042.2
(22) Anmeldetag: 26.02.1997
(51) Int. Cl.: C07C 219/06, C07C 219/08, C11D 1/62

(54) **NIEDRIGSCHMELZENDE ESTERQUATS**
LOW-MELTING ESTERQUATS
COMPOSES QUATERNAIRES D'ESTER A FAIBLE POINT DE FUSION

(30) Priorität: 01.03.1996 DE 19607824
(43) Veröffentlichungstag der Anmeldung: 07.01.1999
(73) Patentinhaber: Clariant GmbH, 65929 Frankfurt am Main (DE)
(72) Erfinder: EYRISCH, Oliver, D-84489 Burghausen (DE); AIGNER, Rudolf, D-84508 Burgkirchen (DE)
(86) Internationale Anmeldenummer: EP9700922
(87) Internationale Veröffentlichungsnummer: WO9731888

(56) Entgegenhaltungen:
- JOURNAL OF BIOPHARMACEUTICAL SCIENCES, Bd. 2, Nr. 1, 1991, Seiten 1-10, XP000675370 FERDINAND DEVINSKY ET AL.: "Structure - Activity Relationships of "Soft" Quaternary Ammonium Amphiphiles"
- RUSSIAN JOURNAL OF APPLIED CHEMISTRY, Bd. 67, Nr. 5, 10.November 1994, NTS BUREAU US, Seiten 734-736, XP002032735 YU. A. RYZHKOV ET AL.: "Synthesis and Thermal Stability of Cationic Surface- Active Dimethylaminoethanol Derivatives"
- CHEMICAL ABSTRACTS, vol. 76, no. 21, 22.Mai 1972 Columbus, Ohio, US; abstract no. 125937m, G. CERBAI ET AL.: "Acetylene derivatives with Antispastic Activity. II. Amino Esters of Propylpropargylacetic acid" Seite 413; XP002032737 & FARMACO, ED. SCI., Bd. 27, Nr. 3, 1972, Seiten 217-234,
- ARZNEIMITTEL FORSCHUNG DRUG RESEARCH., Bd. 45, Nr. 2, 1995, AULENDORF DE, Seiten 198-199, XP002032736 V. MAJTAN ET AL.: "Efficacy of New Organic Ammonium Salts on Pseudomonas aeruginosa and Salmonella typhimurium"
- CHEMICAL ABSTRACTS, vol. 108, no. 24, 13.Juni 1988 Columbus, Ohio, US; abstract no. 206712r, ALOIS NOVACEK ET AL.: "Preparation of Cationic Surfactants as Antistatic Agents for textiles" Seite 119; Spalte 2; XP002032738 in der Anmeldung erwähnt & CS 246 133 B (ALOIS NOVACEK ET AL.)

## Beschreibung

Die Erfindung betrifft niedrigschmelzende quartäre Carbonsäureethanolaminester-Chloride, ein Verfahren zu ihrer Herstellung und ihre Verwendung.

Quartäre Carbonsäureethanolaminester-Salze, auch als Esterquats bezeichnet, stellen hochwirksame und vielseitig verwendbare kationische Tenside dar. So sind diese Tenside zum Beispiel als Wäscheweichspüler, Kosmetikgrundstoffe, Wirkstoffe bezüglich Soil-Release und Soil-Redeposition, Antistatikmittel, Avivagemittel, Biozid und Phasentransferkatalysatoren geeignet. Da diese Esterquats aufgrund ihrer biologischen Abbaubarkeit auch ökologisch vorteilhaft sind, haben sie in letzter Zeit die klassischen Fettalkylquats wie Distearyldimethylammoniumchlorid weitgehend abgelöst.

Hinsichtlich Lagerung, Transport und Weiterverarbeitung sind solche Esterquats erwünscht, die schon bei Raumtemperatur flüssig sind oder zumindest einen niedrigen Schmelzpunkt aufweisen. Sie sollten ferner keine toxischen Verbindungen enthalten und insbesondere im Hinblick auf Weiterverarbeitung zu fertigen Formulierungen auch gut wasserlöslich oder wasserdispergierbar sein. Erwünscht ist darüber hinaus eine möglichst hohe Reinheit.

In DE-A-37 10 064 werden quaternäre Esteramine in Form von Halogeniden und Sulfaten beschrieben, die einen niedrigen Schmelzpunkt aufweisen, da sie schon bei Raumtemperatur eine pastöse (wachsartige) Konsistenz haben. Der niedrige Schmelzpunkt wird jedoch nur dadurch erreicht, daß diese Esterquats eine mehr oder weniger große Menge an Glycerid oder Partialglycerid enthalten. Das Quaternisierungsmittel ist ein Alkylhalogenid wie Methylchlorid oder ein Alkylsulfat wie Dimethylsulfat. Im letzteren Falle kann das Esterquat-Glycerid-Gemisch auch toxisches Dialkylsulfat enthalten. Die Quaternisierung wird ausweislich aller Beispiele in Gegenwart eines organischen Lösungsmittels wie Isopropanol durchgeführt.

In US-A-5 463 094 werden quaternäre Ammoniumsulfate und Esteraminsulfate beschrieben. Ihre Herstellung erfolgt in Abwesenheit von Lösungsmitteln. Das Quaternisierungsmittel ist das an sich vorteilhafte (reaktionsfähige) Dimethylsulfat. Zur Vermeidung von toxischem Restdialkylsulfat im erhaltenen Esterquat, wird das Dialkylsulfat in einer unterstöchiometrischen Menge eingesetzt. Daraus resultiert aber, daß das Esterquat eine mehr oder weniger große Menge an Ausgangsverbindung enthält. Selbst wenn ihre Abtrennung und die Gewinnung von reiner Esterquat-Verbindung möglich ist, wäre dies aufwendig und kostspielig.

Schließlich seien noch CS-B-246 133 und CS-B-264 073 genannt, in denen ebenfalls Ethanolaminestersulfate beschrieben werden. Auch diese jeweils mit Dimethylsulfat hergestellten Esterquats weisen die oben erwähnten Nachteile auf.

Es wurden nun Esterquat-Verbindungen gefunden, die einen niedrigen Schmelzpunkt auch ohne Mithilfe weiterer Komponenten aufweisen. Sie sind darüber hinaus gut wasserlöslich und enthalten aufgrund ihrer Herstellung keine toxischen Anteile. Die erfindungsgemäßen Esterquat-Verbindungen entsprechen der nachstehenden Formel (1) 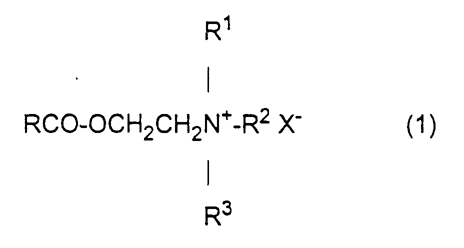 worin
- RCO: ein Fettacylrest mit 6 bis 22 Kohlenstoffatomen, vorzugsweise 8 bis 18 Kohlenstoffatomen,
- R¹: ein Methylrest ist,
- R² und R³: gleich sind und einen C₃-C₆-Alkylrest bedeuten und
- X⁻: Chloridanion ist.

Als besonders bevorzugte Esterquats der Formel (1) haben sich jene herausgestellt, wobei RCO der genannte Acylrest und X⁻ das genannte Anion ist und R¹, R² und R³ für die folgenden Akylreste stehen:
R¹ Methyl, R² Propyl, R³ Propyl (Esterquat 1c) und
R¹ Methyl, R² Butyl, R³ Butyl (Esterquat 1d).

Die für R¹, R² und R³ genannten Alkylreste können gesättigt oder ungesättigt, gerade oder verzweigt sein, wobei gesättigt und gerade bevorzugt ist. Der Rest RCO- ist ein Fettacylrest mit der genannten Anzahl von Kohlenstoffatomen. Er kann gesättigt oder ungesättigt (vorzugsweise ein- bis dreifach ungesättigt) sein. Als Beispiele seien die Acylreste von Capryl-, Caprin-, Laurin-, Palmitin-, Stearin- und Ölsäure genannt sowie Cocosacyl, Talgacyl, vorzugsweise gehärtetes Talgacyl, und dergleichen. Der Fettsäurerest stellt häufig eine Mischung von zwei oder mehreren Acylgruppen dar, zum Beispiel C₁₂ und C₁₄-Acyl (C_{12/14}), C₁₆ und C₁₈-Acyl (C_{16/18}) oder C₁₂ bis C₁₈-Acyl.

Das erfindungsgemäße Verfahren zur Herstellung der Esterquats der Formel (1) ist dadurch gekennzeichnet, daß man eine Carbonsäureethanolaminester-Verbindung der nachstehenden Formel (2) 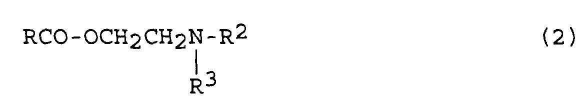 worin RCO, R² und R³ die angegebenen Bedeutungen haben,
mit Methylchlorid in Substanz oder in Gegenwart von Wasser oder einer Mischung von Wasser und niedrigen Alkanolen als Lösungsmittel quaternisiert.

Beim erfindungsgemäßen Verfahren wird also die Quaternisierung unter Verwendung der genannten Lösungsmittel oder in Substanz (das heißt in Abwesenheit von irgendwelchen Lösungsmitteln) und mit Methylchlorid als Quaternisierungsmittel durchgeführt.

Nach einer bevorzugten Verfahrensweise wird in einem ersten Schritt die Veresterung durchgeführt und anschließend das erhaltene Veresterungsprodukt quaternisiert. Ein bevorzugtes erfindungsgemäßes Verfahren ist also dadurch gekennzeichnet, daß man
a) eine Ethanolamin-Verbindung der nachstehenden Formel (3) 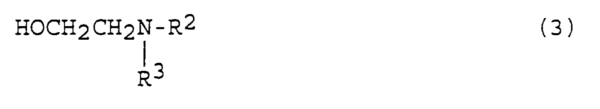 worin R² und R³ die genannten Bedeutungen haben,
   mit einer Carbonsäure der nachstehenden Formel (4)

   RCO-OH (4)

   worin RCO die genannte Bedeutung hat, in Substanz zur Carbonsäureethanolaminester-Verbindung verestert und
b) das im Schritt a) erhaltene Veresterungsprodukt wie oben beschrieben quaternisiert.

Im folgenden wird das erfindungsgemäße Verfahren im einzelnen beschrieben: Die einzusetzenden speziellen Ethanolamine der Formel (3) oder Ethanolaminester der Formel (2) ergeben sich aus den bei Formel (1) angegebenen Bedeutungen für R², R³ und RCO. Das gleiche gilt für die einzusetzende Carbonsäure (Fettsäure) der Formel (4). Die Umsetzung von Ethanolamin-Verbindung und Carbonsäure zur Esterverbindung wird in Substanz, das heißt in Abwesenheit von organischen oder sonstigen Lösungsmitteln, durchgeführt. Die Temperatur der Veresterungsreaktion liegt bei 100 bis 250 °C, vorzugsweise 130 bis 200 °C. Die Reaktionskomponenten Ethanolamin der Formel (3) und Carbonsäure der Formel (4) werden im Molverhältnis von 0,8 bis 1,2 mol Carbonsäure, vorzugsweise 1 bis 1,05 mol Carbonsäure, pro mol Ethanolamin eingesetzt. Zur Beschleunigung der Veresterungsreaktion können Veresterungskatalysatoren eingesetzt werden. Bevorzugt sind saure Katalysatoren, und zwar Halogenwasserstoffsäuren wie Salzsäure; Phosphorsäuren wie unterphosphorige Säure oder Orthophosphorsäure; Schwefelsäure und Sulfonsäuren wie Methansulfonsäure, Paratoluolsulfonsäure-oder Dodecylbenzolsulfonsäure. Bevorzugt sind Phosphorsäuren und Sulfonsäuren. Die Menge an saurem Katalysator beträgt im allgemeinen 0,05 bis 0,5 Gew.-%, bezogen auf das Gewicht des eingesetzten Ethanolamins. Je nach Reaktionstemperatur und Art der Reaktionskomponenten wird die Umsetzung drucklos oder unter dem einsetzenden Druck ablaufen. Es ist bevorzugt, während der Umsetzung eine Inertgasatmosphäre, zum Beispiel Stickstoffatmosphäre, zu halten. Es ferner bevorzugt, das Reaktionswasser aus dem Reaktionsgemisch zu entfernen, zum Beispiel mit Hilfe eines Inertgasstromes und/oder Vakuum. Die Veresterungsreaktion wird zweckmäßigerweise durch gaschromatographische Analyse oder Bestimmung der Säurezahl verfolgt. Die Reaktionszeit liegt im allgemein im Bereich von 5 bis 15 Stunden. Das erhaltene Veresterungsprodukt, das gegebenenfalls mit Wasser gewaschen wird, ist bei Raumtemperatur flüssig bis wachsartig und besteht im wesentlichen aus der angestrebten Carbonsäureethanolaminester-Verbindung gemäß Formel (2).

Die Quaternisierungsreaktion des erfindungsgemäßen Verfahrens wird bevorzugt an dem mit der oben beschriebenen Veresterungsreaktion erhaltenen Esteramin-Produkt durchgeführt. Es können auch auf anderen Wegen erhaltene oder im Handel erhältliche Esteramin-Produkte der Formel (2) eingesetzt werden. Die Quaternisierung mit Methylchlorid wird in Substanz oder in Gegenwart der genannten Lösungsmittel durchgeführt, wobei im ersten Fall (Umsetzung in Substanz) eine Temperatur von 50 bis 200 °C, vorzugsweise 60 bis 150 °C, und im zweiten Fall (Umsetzung in Gegenwart eines Lösungsmittels) eine Temperatur von 40 bis 100 °C, vorzugsweise 50 bis 80 °C, aufrechterhalten wird. In der Wasser/Alkanol-Mischung als Lösungsmittel kann der Anteil an Alkanol innerhalb weiter Grenzen variieren. Er liegt im allgemeinen bei 0 bis 70 Gew.-% Alkanol, vorzugsweise 3 bis 20 Gew.-%, Gewichtsprozente bezogen auf die Mischung aus Wasser und Alkanol. Als niedrige Alkanole sind die C₁ bis C₄-Alkanole wie Methanol, Ethanol, Propanol und Isopropanol bevorzugt. Wie ersichtlich kann die Quaternisierung aufgrund der resultierenden niedrigschmelzenden Esterquats bei relativ niedrigen Temperaturen durchgeführt werden. Aufgrund der weiteren speziellen Eigenschaft dieser strukturell ausgewählten Verbindungen, nämlich ihrer guten Wasserlöslichkeit, kann die Quaternisierung auch bei Verwendung von Wasser allein als Lösungsmittel mit konzentrierten bis hochkonzentrierten wäßrigen Produktmassen durchgeführt werden. So ist es möglich, diese Quaternisierung mit einer Lösungsmittelmenge (zum Beispiel Wassermenge) von weniger als 45 Gew.-% durchzuführen, vorzugsweise 5 bis 25 Gew.-%, Gewichtsprozente bezogen auf die Lösung. Das zu quaternisierende Esteramin als solches oder in Form der genannten Lösungen wird mit gasförmigem Methylchlorid zusammengebracht, wobei das Methylchlorid in einer solchen Menge eingesetzt wird (vor allem aus Sicherheitsgründen), daß ein Druck von maximal 10 bar vorliegt, vorzugsweise von 2 bis 8 bar. Es ist zweckmäßig, die Quaternisierungsreaktion durch laufende Ermittlung des Quaternisierungsgrades zu verfolgen. Die Reaktionszeit liegt im allgemeinen bei 5 bis 15 Stunden. Das Ende der Quaternisierung ist am nicht mehr abnehmenden und konstant bleibenden Druck ersichtlich. Das überschüssige Methylchlorid wird einfach durch Entspannen und gegebenenfalls Evakuieren entfernt. Die erhaltenen, bei Raumtemperatur wachsartigen Esterquats haben einen Schmelzpunkt von höchstens 80 °C, im allgemeinen von höchstens 70 °C, wobei sie gut fließbar (gießbar) sind.

Das erfindungsgemäße Verfahren kann sowohl im Chargenbetrieb als auch kontinuierlich durchgeführt werden. Die kontinuierliche Verfahrensweise wird bevorzugt in mindestens zwei, vorzugsweise zwei bis drei, kaskadenförmig angeordneten Rührkesseln durchgeführt. Dabei ist es vorteilhaft, dem ersten Kessel, nachdem ein Umsatz von Esteramin und Methylchlorid von etwa 10 bis 30 Mol-%, bezogen auf Esteramin, erreicht ist, kontinuierlich Esteramin und Methylchlorid zuzuführen und die Verweilzeit der Produktmasse in den Rührkesseln so einzustellen, daß das Produkt aus dem letzten Kessel den gewünschten Quaternisierungsgrad aufweist.

Die erfindungsgemäße Quaternisierung mit Methylchlorid hat gegenüber jener mit einem Dialkylsulfat den großen Vorteil, daß das Quaternisierungsmittel problemlos auch im Überschuß eingesetzt werden kann, da Methylchlorid im Gegensatz zu Dialkylsulfaten leicht und quantitativ aus dem Reaktionsprodukt entfernbar ist. Mit dem problemlos einsetzbaren Methylchlorid-Überschuß wird als weiterer Vorteil eine erhöhte Reaktionsgeschwindigkeit und ein hoher Umsatzgrad erreicht. Die erfindungsgemäßen quartären Esteraminchloride weisen einen unerwartet niedrigen Schmelzpunkt auf, und zwar auch in Abwesenheit von schmelzpunkterniedrigenden Hilfssubstanzen, Zusätzen, Lösungsmitteln und dergleichen. Sie zeigen ferner gute Wasserlöslichkeit und biologische Abbaubarkeit und können aufgrund ihrer speziellen Herstellungsweise in hoher Reinheit und frei von toxischen Anteilen und organischen Lösungsmittelresten erhalten werden. Die neuen niedrigschmelzenden kationischen Tenside können auch als hochkonzentrierte wäßrige Lösungen (zum Beispiel sogar mit 90 Gew.-% Wirkstoff) zur Verfügung stehen, die trotz der hohen Wirkstoffkonzentration schon bei Raumtemperatur (15 bis 25 °C) gut fließbar sind. Aufgrund dieser hervorragenden Eigenschaften eignen sich die erfindungsgemäßen Esterquats mit besonderem Vorteil überall dort, wo kationische Tenside erwünscht sind, zum Beispiel in allen eingangs genannten Anwendungsbereichen. Ihre erfindungsgemäße Verwendung liegt vorzugsweise in der Bereitung von wäßrigen Formulierungen mit hoher tensidischer Wirkung. Die neuen Esterquats eignen sich vorteilhaft auch zur Bereitung von festen Formulierungen. Dabei geht man vom Schmelzzustand der erfindungsgemäßen Produkte oder von deren Lösungen aus und unterwirft die Schmelze oder die Lösungen üblichen Konfektionierungsverfahren zur Überführung des Produktes in Festform wie Pulver, Pellets, Granulat und dergleichen. Solche Verfahren sind zum Beispiel Sprühkühlung oder Sprühgranulierung im Falle von Schmelzen und Agglomerierung oder Sprühtrocknung im Falle von Lösungen. Zur Konfektionierung können gegebenenfalls Hilfsstoffe eingesetzt werden.

Die Erfindung wird nun anhand von Beispielen und Vergleichsbeispielen noch näher erläutert.

### Beispiel 1

### N,N-Dipropyl-N-methyl-[1-oxododecyl(tetradecyl)oxyethyl]-ammoniumchlorid

In einem Dreihalskolben mit Thermometer, Rührer und Destillationsbrücke werden 612 g (3 mol) Laurinsäure und 445 g (3 mol) Dipropylethanolamin vorgelegt und unter Stickstoffspülung auf 160 °C Innentemperatur aufgeheizt. Nach 2 Stunden Nachreaktionszeit bei 160 °C wird innerhalb einer Stunde auf 190 °C aufgeheizt. Während weiteren 10 Stunden Rühren bei 190 °C destilliert restliches Reaktionswasser über. Man erhält auf diese Weise 1003 g eines gelblichen Öls (Aminzahl 29,8 ml 0,1 N HCl/g).

204 g (0,6 mol) des so erhaltenen Esteramins werden in einem 1-Liter-Druck-Glasautoklav vorgelegt. Der verschlossene Autoklav wird auf 120 °C aufgeheizt und anschließend portionsweise gerade soviel Methylchlorid zudosiert, daß ein Druck von 5 bar nicht überschritten wird. Nach 7 Stunden Rühren bei 120 °C und 4 bis 5 bar wird entspannt und physikalisch gelöstes Methylchlorid durch Evakuieren (200 mbar) entfernt. Man erhält auf diese Weise 234 g der gewünschten quartären Ammoniumverbindung in Form eines schwach gelblichen Feststoffs (Schmelzpunkt 69 °C, kationische Aktivsubstanz 2,53 mmol N/g, Reinheit 95 %).

### Beispiel 2

### N,N-Dibutyl-N-methyl-[1-oxododecyl(tetradecyl)oxyethyl]-ammoniumchlorid

In einem Dreihalskolben mit Thermometer, Rührer und Destillationsbrücke werden 172 g (0,85 mol) Laurinsäure und 145 g (0,85 mol) Dibutylethanolamin vorgelegt und unter Stickstoffspülung auf 160 °C Innentemperatur aufgeheizt. Nach 1 Stunde Nachreaktionszeit bei 160 °C wird innerhalb von 30 Minuten auf 190 °C aufgeheizt. Während weiteren 8 Stunden Rühren bei 190 °C destilliert restliches Reaktionswasser über. Man erhält auf diese Weise 302 g eines gelblichen Öls (Aminzahl 27,6 ml 0,1 N HCl/g).

249 g (0,69 mol) des so erhaltenen Esteramins werden in einem 1-Liter-Druck-Glasautoklav vorgelegt. Der verschlossene Autoklav wird auf 75 bis 80 °C aufgeheizt und anschließend portionsweise gerade soviel Methylchlorid zudosiert, daß ein Druck von 5 bar nicht überschritten wird. Nach 12 Stunden Rühren bei 80 °C und 4 bis 5 bar wird entspannt und physikalisch gelöstes Methylchlorid durch Evakuieren (200 mbar) entfernt. Man erhält auf diese Weise 283 g der gewünschten quartären Ammoniumverbindung in Form eines schwach gelblichen Feststoffs (Schmelzpunkt 54 °C, kationische Aktivsubstanz 2,37 mmol N/g, Reinheit 95 %).

### Beispiel 3

### N,N-Dipropyl-N-methyl-[1-oxododecyl(tetradecyl)oxyethyl]-ammoniumchlorid

286 g (0,85 mol) des Esteramins aus Beispiel 1 werden zusammen mit 36,5 g Wasser in einem 1-Liter-Druck-Glasautoklav vorgelegt. Der verschlossene Autoklav wird auf 60 °C aufgeheizt und anschließend portionsweise gerade soviel Methylchlorid zudosiert, daß ein Druck von 5 bar nicht überschritten wird. Nach 9 Stunden Rühren bei 60 °C und 4 bis 5 bar wird entspannt und physikalisch gelöstes Methylchlorid durch Evakuieren (200 mbar) entfernt. Man erhält auf diese Weise 361 g der gewünschten quartären Ammoniumverbindung in Form eines schwach gelblichen gießfähigen Sirups (Feststoffgehalt 90 %, Reinheit 93 %).

### Vergleichsbeispiel 1

### N,N,N-Trimethyl-[1-oxododecyl(tetradecyl)oxyethyl]-ammoniumchlorid

In einem Dreihalskolben mit Thermometer, Rührer und Destillationsbrücke werden 1,15 kg (5,6 mol) Laurinsäure vorgelegt, unter Stickstoffspülung auf 160 °C Innentemperatur aufgeheizt und innerhalb von 6 Stunden kontinuierlich 645 g (7 mol) Dimethylethanolamin zugetropft. Nach 2 Stunden Nachreaktionszeit bei 160 °C wird innerhalb von 30 Minuten auf 180 °C aufgeheizt. Während weiteren 3 Stunden Rühren bei 180 °C destilliert restliches Reaktionswasser über. Zur Entfernung des überschüssigen Amins werden noch weitere 3 Stunden bei einem Druck von 30 mbar weitergerührt. Man erhält auf diese Weise 1,56 kg eines gelblichen Öls (Aminzahl 33,3 ml 0,1 N HCl/g).

365 g (1,2 mol) des so erhaltenen Esteramins werden in einem 1-Liter-Druck-Glasautoklav mit 74 g Isopropanol vorgelegt. Der verschlossene Autoklav wird auf 75 bis 80 °C aufgeheizt und anschließend innerhalb von 2 Stunden portionsweise gerade soviel Methylchlorid zudosiert, daß ein Druck von 5 bar nicht überschritten wird. Nach 6 Stunden Rühren bei 80 °C und 4 bis 5 bar wird entspannt und physikalisch gelöstes Methylchlorid am Rotationsverdampfer (80 °C/200 mbar) entfernt. Man erhält auf diese Weise die gewünschte quartäre Ammoniumverbindung als 85%ige Einstellung in Isopropanol in Form eines schwach gelblichen Feststoffs (Schmelzpunkt 53 °C, kationische Aktivsubstanz 2,12 mmol N/g, Reinheit 97 %, bezogen auf den Trockenrückstand). Ein Teil des Produkts wird im Vakuum-Trockenschrank (100 °C/100 mbar) getrocknet. Man erhält ein Pulver, das bei 173 °C unter Zersetzung schmilzt.

### Vergleichsbeispiel 2

### N,N-Diethyl-N-methyl-[1-oxododecyl(tetradecyl)oxyethyl]-ammoniumchlorid

In einem Dreihalskolben mit Thermometer, Rührer und Destillationsbrücke werden 612 g (3 mol) Laurinsäure vorgelegt, unter Stickstoffspülung auf 160 °C Innentemperatur aufgeheizt und innerhalb von 5 Stunden kontinuierlich 439 g (3,75 mol) Diethylethanolamin zugetropft. Nach 2 Stunden Nachreaktionszeit bei 160 °C wird innerhalb von 30 Minuten auf 180 °C aufgeheizt. Während weiteren 8 Stunden Rühren bei 180 °C destilliert restliches Reaktionswasser über. Zur Entfernung des überschüssigen Amins werden noch weitere 3 Stunden bei einem Druck von 20 mbar weitergerührt. Man erhält auf diese Weise 909 g eines gelblichen Öls (Aminzahl 32,4 ml 0,1 N HCl/g).

371 g (1,2 mol) des so erhaltenen Esteramins werden in einem 1-Liter-Druck-Glasautoklav mit 76 g Isopropanol vorgelegt. Der verschlossene Autoklav wird auf 75 bis 80 °C aufgeheizt und anschließend innerhalb von 2 Stunden portionsweise gerade soviel Methylchlorid zudosiert, daß ein Druck von 5 bar nicht überschritten wird. Nach 6 Stunden Rühren bei 80 °C und 4 bis 5 bar wird entspannt und physikalisch gelöstes Methylchlorid am Rotationsverdampfer (80 °C/200 mbar) entfernt. Man erhält auf diese Weise die gewünschte quartäre Ammoniumverbindung als 85%ige Einstellung in Isopropanol in Form eines schwach gelblichen Feststoffs (Schmelzpunkt 53 °C, kationische Aktivsubstanz 2,36 mmol N/g, Reinheit 99 %, bezogen auf den Trockenrückstand). Ein Teil des Produkts wird im Vakuum-Trockenschrank (100 °C/100 mbar) getrocknet. Man erhält ein Pulver, das bei 155 °C unter Zersetzung schmilzt.

### Vergleichsbeispiel 3

### N,N-Dioctyl-N-methyl-[1-oxododecyl(tetradecyl)oxyethyl]-ammoniumchlorid

In einem Dreihalskolben mit Thermometer, Rührer und Destillationsbrücke werden 36,4 g (0,18 mol) Laurinsäure und 49 g (0,18 mol) Dioctylethanolamin vorgelegt und unter Stickstoffspülung auf 160 °C Innentemperatur aufgeheizt. Nach 1 Stunde Nachreaktionszeit bei 160 °C wird innerhalb von 30 Minuten auf 190 °C aufgeheizt. Während weiteren 11 Stunden Rühren bei 190 °C destilliert restliches Reaktionswasser über. Man erhält auf diese Weise 82 g eines gelblichen Öls (Aminzahl 20,7 ml 0,1 N HCl/g).

50 g (0,1 mol) des so erhaltenen Esteramins werden in einem 1-Liter-Druck-Glasautoklav mit 13,8 g Isopropanol vorgelegt. Der verschlossene Autoklav wird auf 75 bis 80 °C aufgeheizt und anschließend innerhalb von 2 Stunden portionsweise gerade soviel Methylchlorid zudosiert, daß ein Druck von 5 bar nicht überschritten wird. Nach 3 Tagen Rühren bei 80 °C und 4 bis 5 bar wird entspannt und physikalisch gelöstes Methylchlorid am Rotationsverdampfer (80 °C/200 mbar) entfernt. Man erhält auf diese Weise die gewünschte quartäre Ammoniumverbindung als 85%ige Einstellung in Isopropanol in Form eines schwach gelblichen Öls (kationische Aktivsubstanz 1,62 mmol N/g, Reinheit 92 %, bezogen auf den Trockenrückstand). Ein Teil des Produkts wird im Vakuum-Trockenschrank (100 °C/100 mbar) getrocknet. Man erhält einen Feststoff, der bei 93 bis 95 °C schmilzt.

### Vergleichsbeispiel 4

### N-Methyl-[1-oxododecyl(tetradecyl)oxyethyl]-morpholiniumchlorid

In einem Dreihalskolben mit Thermometer, Rührer und Destillationsbrücke werden 652 g (3,2 mol) Laurinsäure und 417 g (3,2 mol) Hydroxyethylmorpholin vorgelegt und unter Stickstoffspülung auf 160 °C Innentemperatur aufgeheizt. Nach 1 Stunde Nachreaktionszeit bei 160 °C wird innerhalb von 30 Minuten auf 190 °C aufgeheizt. Während weiteren 29 Stunden Rühren bei 190 °C destilliert restliches Reaktionswasser über. Man erhält auf diese Weise 1012 g eines gelblichen Öls (Aminzahl 30,8 ml 0,1 N HCl/g).

195 g (0,6 mol) des so erhaltenen Esteramins werden in einem 1-Liter-Druck-Glasautoklav mit 72 g Isopropanol vorgelegt. Der verschlossene Autoklav wird auf 75 bis 80 °C aufgeheizt und anschließend innerhalb von 2 Stunden portionsweise gerade soviel Methylchlorid zudosiert, daß ein Druck von 5 bar nicht überschritten wird. Nach 38 Stunden Rühren bei 80 °C und 4 bis 5 bar wird entspannt und physikalisch gelöstes Methylchlorid am Rotationsverdampfer (80 °C/200 mbar) entfernt. Man erhält auf diese Weise die gewünschte quartäre Ammoniumverbindung als 80%ige Einstellung in Isopropanol in Form eines schwach gelblichen Öls (kationische Aktivsubstanz 2,05 mmol N/g, Reinheit 94 %, bezogen auf den Trockenrückstand). Ein Teil des Produkts wird im Vakuum-Trockenschrank (100 °C/100 mbar) getrocknet. Man erhält einen Feststoff, der über 150 °C schmilzt.

## Patentansprüche

1. Niedrigschmelzende quartäre Carbonsäureethanolaminester Chloride, **gekennzeichnet durch** die nachstehende Formel (1) 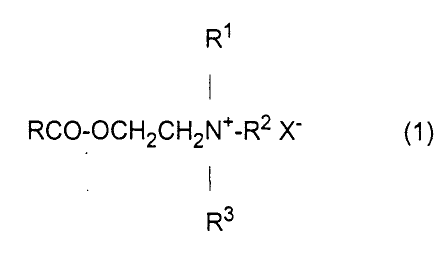 worin
RCO ein Fettacylrest mit 6 bis 22 Kohlenstoffatomen,
R¹ ein Methylrest ist,
R² und R³ gleich sind und einen C₃ bis C₆-Alkylrest bedeuten und
X⁻ ein Chloridanion ist.

2. Quartäre Verbindungen nach Anspruch 1, **dadurch gekennzeichnet, daß** in Formel (1) RCO ein Fettacylrest mit 6 bis 22 Kohlenstoffatomen und X⁻ ein Chloridanion ist und R¹, R² und R³ für die folgenden Alkylreste stehen:
R¹ Methyl, R² Propyl, R³ Propyl oder für
R¹ Methyl, R² Butyl, R³ Butyl.

3. Verfahren zur Herstellung der quartären Carbonsäureethanolaminester-Chloride nach Anspruch 1, **dadurch gekennzeichnet, daß** man Carbonsäureethanolaminester der nachstehenden Formel (2) 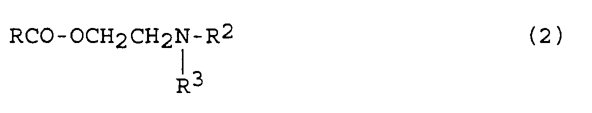 worin RCO, R² und R³ die angegebenen Bedeutungen haben,
mit Methylchlorid in Substanz oder in Gegenwart von Wasser oder einer Mischung von Wasser und niedrigen Alkanolen als Lösungsmittel quaternisiert.

4. Verfahren nach Anspruch 3, **dadurch gekennzeichnet, daß** man
a) Ethanolamine der nachstehenden Formel (3) 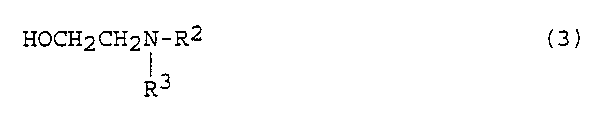 worin R² und R³ die genannten Bedeutungen haben,
mit einer Carbonsäure der nachstehenden Formel (4)
RCO-OH (4)
worin RCO die genannte Bedeutung hat, in Substanz zur Carbonsäureethanolaminester-Verbindung verestert und
b) das im Schritt a) erhaltene Veresterungsprodukt mit Methylchlorid in Substanz oder in Gegenwart von Wasser oder einer Mischung von Wasser und niedrigen Alkanolen als Lösungsmittel quaternisiert.

5. Verfahren nach Anspruch 3 oder 4, **dadurch gekennzeichnet, daß** man die Quaternisierung in Gegenwart von Wasser und 0 bis 70 Gew.-% von einem niedrigen Alkanol als Lösungsmittel, Gewichtsprozente bezogen auf die Mischung aus Wasser und Alkanol, und mit einer Lösungsmittelmenge von weniger als 45 Gew.-%, bezogen auf die Produktlösung, durchführt.

6. Verfahren nach einem oder mehreren der Ansprüche 3 bis 5, **dadurch gekennzeichnet, daß** man die Quaternisierung in Substanz bei einer Temperatur von 50 bis 200 °C und die Quaternisierung in Gegenwart von Lösungsmitteln einer Temperatur von 40 bis 100 °C durchführt.

7. Verfahren nach einem oder mehreren der Ansprüche 3 bis 6, **dadurch gekennzeichnet, daß** man die Quaternisierung kontinuierlich in mindestens zwei kaskadenförmig angeordneten Rührkesseln durchführt.

8. Verwendung der Carbonsäureethanolaminester-Chloride nach Anspruch 1 zur Bereitung von festen oder wäßrigen Formulierungen mit hoher tensidischer Wirkung.

## Claims

1. A low-melting quaternary carboxylic acid ethanolamine ester chloride, **characterized by** the formula (1) below 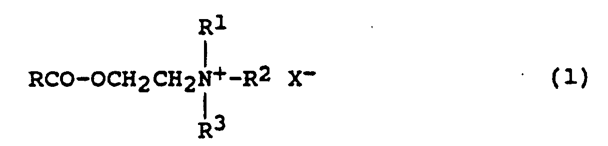 where
RCO is a fatty radical having 6 to 22 carbon atoms,
R¹ is a methyl radical,
R² and R³ are identical and are each a C₃-C₆-alkyl radical and
X⁻ is a chloride anion.

2. A quaternary compound as claimed in claim 1, wherein, in formula (1), RCO is a fatty acyl radical having 6 to 22 carbon atoms and X⁻ is a chloride anion and R¹, R² and R³ are the following alkyl radicals:
R¹ methyl, R² propyl, R³ propyl or
R¹ methyl, R² butyl, R³ butyl.

3. A process for preparing the quaternary carboxylic acid ethanolamine ester chloride as claimed in claim 1, which comprises quaternizing a carboxylic acid ethanolamine ester of the formula (2) below 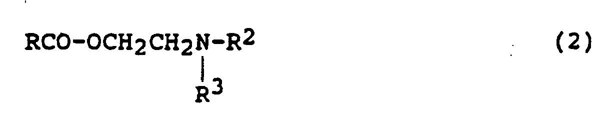 where RCO, R² and R³ have the specified meanings, with methyl chloride in the absence of solvents or in the presence of water or a mixture of water and low alkanols as solvent.

4. The process as claimed in claim 3, wherein
a) ethanolamines of the formula (3) below 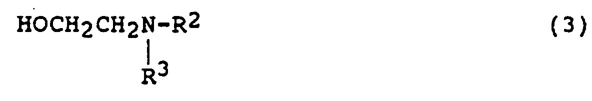 where R² and R³ have said meanings, are esterified with a carboxylic acid of the formula (4) below
RCO-OH (4)
where RCO has said meaning, in the absence of solvents to give the carboxylic acid ethanolamine ester compound and
b) the esterification product obtained in step a) is quaternized with methyl chloride in the absence of solvents or in the presence of water or a mixture of water and low alkanols as solvent.

5. The process as claimed in claim 3 or 4, wherein the quaternization is carried out in the presence of water and 0 to 70% by weight of a low alkanol as solvent, percentages by weight based on the mixture of water and alkanol, and using an amount of solvent of less than 45% by weight, based on the product solution.

6. The process as claimed in one or more of claims 3 to 5, wherein the quaternization is carried out in the absence of solvents at a temperature of 50 to 200°C and the quaternization in the presence of solvents is carried out at a temperature of 40 to 100°C.

7. The process as claimed in one or more of claims 3 to 6, wherein the quaternization is carried out continuously in at least two stirred tanks arranged in cascade.

8. The use of the carboxylic acid ethanolamine ester chlorides as claimed in claim 1 to prepare solid or aqueous formulations having a high surfactant effect.

## Revendications

1. Chlorures d'esters quaternaires à bas point de fusion d'éthanolamines et d'acides carboxyliques, **caractérisés par** la formule (1) ci-après : 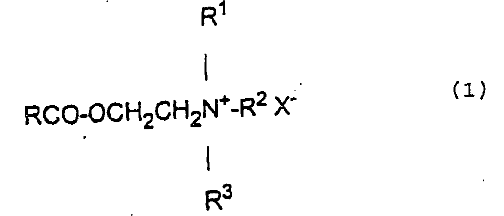 dans laquelle
RCO est un radical acyle gras ayant de 6 à 22 atomes de carbone,
R¹ est un radical méthyle,
R² et R³ sont identiques et représentent chacun un radical alkyle en C₃ à C₆, et
X⁻ est un anion chlorure.

2. Composés quaternaires selon la revendication 1, **caractérisés en ce que**, dans la formule (1), RCO est un radical acyle gras ayant de 6 à 22 atomes de carbone, et X⁻ est un anion chlorure, et R¹, R² et R³ représentent les radicaux alkyle suivants :
R¹ méthyle, R² propyle, R³ propyle, ou
R¹ méthyle, R² butyle, R³ butyle.

3. Procédé de préparation des chlorures d'esters quaternaires d'éthanolamines et d'acides carboxyliques selon la revendication 1, **caractérisé en ce qu'**on quaternise des esters d'éthanolamines et d'acides carboxyliques ayant la formule (2) ci-après : 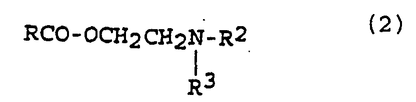 dans laquelle RCO, R² et R³ ont les significations données ci-dessus,
avec du chlorure de méthyle, sans solvant ou en présence, en tant que solvant, d'eau ou d'un mélange d'eau et d'alcanols inférieurs.

4. Procédé selon la revendication 3, **caractérisé en ce que**
a) on estérifie des éthanolamines ayant la formule (3) ci-après : 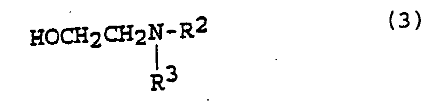 dans laquelle R² et R³ ont les significations ci-dessus,
avec un acide carboxylique ayant la formule (4) ci-après :
RCO-OH (4)
dans laquelle RCO a la signification donnée ci-dessus,
en l'absence de solvant pour donner le composé ester d'éthanolamine et d'acide gras, et
b) on quaternise le produit d'estérification obtenu dans l'étape a), à l'aide de chlorure de méthyle, sans solvant ou en présence, en tant que solvant, d'eau ou d'un mélange d'eau et d'alcanols inférieurs.

5. Procédé selon la revendication 3 ou 4, **caractérisé en ce qu'**on met en oeuvre la quaternisation en présence, en tant que solvant, d'eau et de 0 à 70 % en poids d'un alcanol inférieur, les pourcentages en poids étant rapportés au mélange d'eau et d'alcanol, et avec une quantité de solvant inférieure à 45 % en poids par rapport à la solution produite.

6. Procédé selon l'une ou plusieurs des revendications 3 à 5, **caractérisé en ce qu'**on met en oeuvre la quaternisation en l'absence de solvant à une température de 50 à 200°C, et la quaternisation en présence de solvants à une température de 40 à 100°C.

7. Procédé selon l'une ou plusieurs des revendications 3 à 6, **caractérisé en ce qu'**on met en oeuvre la quaternisation en continu dans au moins deux cuves à agitation disposées en cascade.

8. Utilisation des chlorures d'esters d'éthanolamines et d'acides carboxyliques selon la revendication 1 pour prépa-rer des formulations solides ou aqueuses ayant un effet tensioactif élevé.
